# EUROPEAN PATENT APPLICATION

(11) **EP 0 768 070 A1**
(43) Date of publication of application: **16.04.1997**
(21) Application number: 95116270.0
(22) Date of filing: 16.10.1995
(51) Int. Cl.: A61F 13/00, A61F 13/15

(54) **Compound disposable absorbent article with a hump-forming element**

(71) Applicant: THE PROCTER & GAMBLE COMPANY, Cincinnati, Ohio 45202 (US)
(72) Inventor: Wierlacher, Stefan Alois, D-60528 Frankfurt (DE); Plumley, Julian Ashton, D-74589 Satteldorf 1 (DE)
(74) Representative: Hirsch, Uwe Thomas

(57) **Abstract**

A compound disposable absorbent article for wearing adjacent a body discharge area having a body facing surface, a garment facing surface, a longitudinal axis, a lateral axis, a primary absorbent member with a length and a width and a secondary absorbent member with a length and a width. The primary absorbent member and the secondary absorbent member have their length each parallel to the longitudinal axis. The secondary absorbent member is closer to the garment facing surface than the primary absorbent member. The primary absorbent member is affixed to the secondary absorbent member by joining means. The width of the secondary absorbent member is preferably equal or greater than the width of the primary absorbent member. The article further comprises a yielding fluid bubble to raise the body facing surface towards the discharge area.

## Description

### FIELD OF THE INVENTION

The present invention relates to compound disposable absorbent articles. Disposable absorbent articles are considered to be absorbent devices designed to be worn externally of the body by a user and to receive fluids discharged from the body. In particular the present invention relates to compound disposable absorbent sanitary napkins, catamenials, incontinence inserts and pantiliners comprising a fluid bubble to raise the body facing surface of the article towards the wearer especially in the area of liquid discharge. The fluid bubble is filled with gas such that it yields to external pressures in order to maintain a comfortable fit of the absorbent article.

### BACKGROUND OF THE INVENTION

In their simplest form, disposable absorbent articles comprise an absorbent element (sometimes referred to as an absorbent core) interposed between a pervious body-contacting element (sometimes referred to as a topsheet or an overwrap) and an impervious protective barrier (sometimes referred to as a backsheet). The absorbent element is, of course, intended to receive and contain the fluids discharged from the body. The body-contacting element is intended to provide more or less comfortable and dry-feeling contact with body surfaces while allowing free passage of fluids therethrough into the absorbent element. The protective barrier is intended to prevent the fluids which are expelled or which escape from the absorbent element from soiling the user's garments.

In addition to the three functional elements mentioned above, disposable absorbent articles are generally provided with means for supporting the device adjacent the user's crotch area, even as the user moves, where it can most effectively perform its intended function. Typically, absorbent articles as sanitary napkins are provided with an adhesive attachment means for securing the device to the inner crotch area of the user's undergarments.

While previously known absorbent articles do perform their intended function, each conventional design suffers from certain deficiencies in one or more of absorbency of body fluids, protection of the user's garments from soiling, and/or physical comfort to the user.

With respect to disposable sanitary napkins, at least two general classes presently exist. One such class is identified as being intended for the absorption of medium to high menstrual flows. These sanitary napkins offer a relatively high absorptive capacity. Absorptive capacity is commonly achieved by providing the sanitary napkin with a relatively thick and bulky absorbent member. While having a relatively high absorptive capacity, the bulkiness of the absorbent member may cause a certain degree of wearing discomfort.

A second class of sanitary napkins are intended for light or low menstrual flows and are commonly referred to as pantiliners or pantishields. Sanitary napkins of this class, as a group, are thinner, somewhat more flexible and generally more comfortable than those of the first class. However, sanitary napkins of the second class typically lack the absorptive capacity of sanitary napkins of the first class.

One attempt to provide the benefits of the previously described two classes of sanitary napkins into a single compound sanitary napkin is disclosed in U.S. Patent No. 4,425,130. This compound sanitary napkin comprises a primary menstrual pad and a panty protector joined to one another at their corresponding ends in such a manner that the two constituents are free to move relative to one another along essentially their entire common length. The primary menstrual pad is intended to absorb the bulk of the bodily fluids discharged by the user, while the panty protector is intended to protect the user's garments from soiling. In use, the relative freedom of movement between the primary menstrual pad and the panty protector serves to maintain the primary menstrual pad adjacent the user's crotch region while the panty protector remains associated with the user's undergarment. While the relative freedom of movement between the primary menstrual pad and the panty protector serves to maintain the primary menstrual pad near the user's crotch region, this freedom of movement may lead to a lack of stability if the primary menstrual pad moves laterally beyond the side edges of the panty protector, providing an opportunity for soiling the user's undergarment.

Moreover, the bulky primary menstrual pad, though capable of providing a close body contact, is not sufficiently flexible to mold and conform to the anatomy of the user, and therefore may cause discomfort.

Another attempt to combine the benefits of the previously described two classes of absorbent articles is disclosed in US application 08/294,663 filed on 19 August 1994 and assigned to Mayer et al. The self adapting compound sanitary napkin described in this application comprises a primary absorbent member having a length and a width and a secondary absorbent member having a length and a width. The primary absorbent member and the secondary absorbent member have a common length. The primary absorbent member includes an absorbent core and a fluid pervious topsheet superimposed on said absorbent core. The secondary absorbent member includes a fluid pervious topsheet, a fluid impervious backsheet joined to said topsheet and an absorbent element positioned between the topsheet and the backsheet. The primary absorbent member is affixed to the secondary absorbent member by union means. The width of the secondary absorbent member is preferably at least 1.5 times the width of the primary absorbent member.

While this type of design is effective in that it arranges most of the absorbent material along the centre-line of the pad, where it can absorb promptly the bulk of bodily fluids discharged by the user, it still can have the disadvantage of a poor fit to the anatomy . In an attempt to solve this problem the primary absorbent member may be made bulky enough to stay in intimate contact with the body of the user, or, alternatively, a resilient member may be optionally comprised within the primary absorbent member itself; the resilient member may be constituted by e.g. a fibrous material, or by a hollow and resilient structure.

In any case the structures described above can have the disadvantage of being uncomfortable to the user since they are not capable of molding and conforming effectively to the anatomy of the user.

### SUMMARY OF THE INVENTION

The present invention relates to a compound disposable absorbent article for wearing adjacent a body discharge area. The compound disposable absorbent article has a body facing surface, a garment facing surface, a longitudinal axis and a lateral axis, a primary absorbent member having a length and a width and a secondary absorbent member having a length and a width. The primary absorbent member and the secondary absorbent members have their length each parallel to the longitudinal axis. The secondary absorbent member is closer to the garment facing surface than the primary absorbent member. The primary absorbent member is affixed to the secondary absorbent member by joining means, and the width of the secondary absorbent member is preferably equal or greater than the maximum width of the primary absorbent member. The compound disposable absorbent article further comprises a yielding fluid bubble, preferably an air bubble, to raise the body facing surface towards the discharge area.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a top plan view of one embodiment of a compound sanitary napkin according to the present invention;
FIG. 2 is a cross-sectional view of the compound sanitary napkin shown in FIG. 1 as taken along section line 2-2;
FIG. 3 is a cross-sectional view of the compound sanitary napkin shown in FIGS. 1 and 2 as taken along section line 3-3 of FIG. 2;
FIGS. 3A is a cross-sectional view of another embodiment of compound sanitary napkin of the present invention;
FIG. 4 is a top plan view of another embodiment of a compound sanitary napkin of the present invention;
FIG. 5 is a top plan view of another embodiment of a compound sanitary napkin of the present invention;
FIG. 6 is a cross-sectional view of another embodiment of a compound sanitary napkin of the present invention;
FIG. 7 is a cross-sectional view of another embodiment of a compound sanitary napkin of the present invention;
FIG. 8 is a cross-sectional view of another embodiment of a compound sanitary napkin of the present invention;
FIG. 9 is a cross-sectional view of another embodiment of a compound sanitary napkin of the present invention;

### DETAILED DESCRIPTION OF THE INVENTION

This invention is of a compound disposable absorbent article which exhibits absorbency for bodily fluids, the protection of the user's garments from soiling, and improved physical comfort to the user. The compound disposable absorbent article is described below by reference to a sanitary napkin or catamenial. The term "sanitary napkin", as used herein, refers to an article which is worn by females adjacent to the pudendal region and which is intended to absorb and contain the various exudates which are discharged from the body (e.g., blood, menses, and urine) and which is intended to be discarded after a single use.

The term "compound sanitary napkin", as used herein, refers to a sanitary napkin comprised of separate constituents joined to one another to form a unitary structure.

The terms "joined" or "affixed", as used herein, encompasses configurations whereby a first member is directly connected to a second member and configurations whereby a first member is indirectly connected to a second member by connecting the first member to intermediate members which in turn are connected to the second member.

Interlabial devices which reside partially within and partially external of the wearer's vestibule are also within the scope of this invention. As used herein, the term "pudendal" refers to the externally visible female genitalia and is limited to the labia majora, the labia minora, the clitoris, and the vestibule.

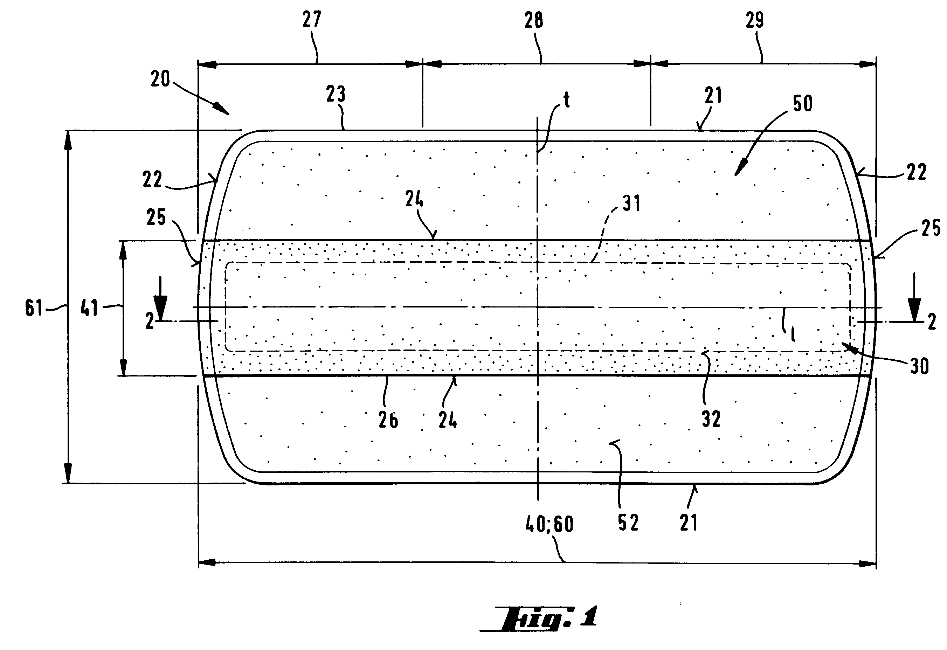

In FIGS. 1-3, one preferred embodiment of a compound sanitary napkin 20 of the present invention is shown. As can be seen in FIGS. 1-3, the compound sanitary napkin 20 comprises a primary absorbent member 30, a secondary absorbent member 50 affixed together by joining means 70 and a fluid bubble 31 comprised within the primary absorbent member 30.

The compound sanitary napkin has two surfaces, a body contacting or facing surface, and a garment facing or contacting surface. The primary and secondary absorbent members each have corresponding body facing and garment facing surfaces. In use, the secondary absorbent member 50 is intended to stay closer to the garment facing surface of the sanitary napkin 20 than the primary absorbent member 30 which, in turn, is in direct contact with the anatomy of the user. The compound sanitary napkin 20 has two axis, a longitudinal axis and a transverse axis. The term "longitudinal", as use herein, refers to a line, axis or direction in the plane of the compound sanitary napkin that is generally aligned with (e.g., approximately parallel to) a vertical plane which bisects a standing wearer into left and right body halves when the compound sanitary napkin is worn. The terms "transverse" or "lateral", as used herein, are interchangeable, and refer, to a line, axis, or direction which lies within the plane of the compound sanitary napkin that is generally perpendicular to the longitudinal direction.

The primary absorbent member 30 has side edges 24 and end edges 25 which together form the periphery 26 of the primary absorbent member. The secondary absorbent member 50 has side edges 21 and end edges 22 which together form the periphery 23 of the secondary absorbent member and the compound sanitary napkin 20. The compound sanitary napkin 30 has a first end region 27, a central region 28, and a second end region 29.

The primary absorbent member 30 is that constituent of the compound sanitary napkin 20 intended to first receive the bodily fluids discharged by the user. The primary absorbent member 30 comprises an absorbent means 33, such as absorbent core 34 and a liquid permeable topsheet or coverstock 32 superimposed on the absorbent core 34; in the embodiment of Fig. 1 to 3 the primary absorbent member 30 further comprises a fluid bubble 31, typically an air bubble, positioned within the absorbent core 34.

The topsheet 32 is compliant, soft feeling, and non-irritating to the wearer's skin. Further, the topsheet 32 is liquid pervious, permitting liquid to readily penetrate through its thickness. A suitable topsheet 32 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibers), synthetic fibers (e.g., polymeric fibers such as polyester, polypropylene, or polyethylene fibers); or from a combination of natural and synthetic fibres.

A preferred topsheet comprises an apertured formed film. Apertured formed films are preferred for the topsheet because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer.

Suitable formed films are described in U.S. Pat. No. 3,929,135, issued to Thompson on December 30, 1975; U.S. Pat. No. 4,324,246, issued to Mullane, et al. on April 13, 1982; U.S. Pat. No. 4,342,314, issued to Radel, et al. on August 3, 1982; U.S. Pat. No. 4,463,045, issued to Ahr, et al. on July 31, 1984; and U.S. Pat. No. 5,006,394, issued to Baird on April 9, 1991. The preferred topsheet for the primary absorbent member of the present invention is a formed film described in one or more of the above patents and marketed on sanitary napkins by The Procter & Gamble Company of Cincinnati, Ohio as "DRI-WEAVE".

In a preferred embodiment of the present invention, the body or exposed surface of the formed film topsheet is hydrophilic so as to help liquid transfer through the topsheet faster than if the body surface was not hydrophilic so as to diminish the likelihood that menstrual fluid will flow off the topsheet rather than flowing into and being absorbed by the absorbent core.

The topsheet 32 may be associated with the absorbent core 34 in any suitable manner. Suitable manners include, but are not limited to associating the topsheet 32 with the absorbent core 34 with adhesives such as by spray-gluing or applying lines or spots of adhesives between the topsheet 32 and the absorbent core 34.

Alternatively, or additionally, the topsheet 32 may be associated with the absorbent core 34 by entangling the fibers of the absorbent core 34 with the topsheet 32, by fusing the topsheet 32 to the absorbent core 34 with a plurality of discrete individual fusion bonds.

To insure proper fluid transfer between the topsheet 32 and the absorbent core 34 it is preferred that the topsheet be substantially continuously secured to the underlying absorbent core 34 throughout their common interface. By substantially continuously securing the topsheet 32 to the underlying absorbent core 34 the topsheet 32 will have a reduced tendency to separate from the absorbent core 34 during use. Separation of the absorbent core from the topsheet 32 may inhibit fluid transfer from the topsheet 32 into the underlying absorbent core 34.

The absorbent core 34 may be any absorbent means which is generally compressible, conformable, resilient, non-irritating to the wearer's skin and capable of absorbing and containing body exudates. The absorbent core 34 may be manufactured from a wide variety of liquid absorbent materials commonly used in disposable sanitary napkins, and other disposable absorbent articles. Examples of suitable absorbent materials include comminuted wood pulp (which is generally referred to as airfelt), creped cellulose wadding, modified cross-linked cellulose fibers (such as those described in U.S. Patent No. 5,217,445 issued to Young, et al. on June 8, 1993), capillary channel fibers (that is, fibers having intra-fiber capillary channels such as those described in U.S. Patent No. 5,200,248 issued to Thompson, et al. on April 6, 1993), absorbent foams (such as those described in U.S. Patent No. 5,260,345, issued to DesMarais, et al. on November 9, 1993 and U.S. Patent No. 5,268,244 issued to DesMarais, et al. on December 7, 1993), thermally bonded airlay materials (such as those material described in U.S. Patent Application Serial No. 08/141,156, entitled "Catamenial Absorbent Structures Having Thermally Bonded Layers For Improved Handling of Menstrual Fluids and Their Use In Catamenial Pads Having Improved Fit and Comfort" filed in the name of Richards, et al. on October 21, 1993 (P&G Case 5051)), absorbent sponges, synthetic staple fibers, polymeric fibers, hydrogel-forming polymer gelling agents, peat moss, or any equivalent materials or combinations of materials. Suitable absorbent cores comprising foams are described in European Applications 0 598 833, 0 598 823 and 0 598 834.

The total absorbent capacity of the absorbent core 34 together with the absorbent capacity of the secondary absorbent member should be compatible with the intended exudate loading for the compound sanitary napkin 20. Further, the absorbent capacity of the absorbent core 34 may be varied to accommodate wearers ranging in the expected amount of exudate fluid volume. For instance, a different absorbent capacity may be utilized for compound sanitary napkins intended for day time use as compared with those intended for night time use, or for compound sanitary napkins intended for use by teenage females as compared with those intended by more mature women.

Materials selected for use as the absorbent core 34 are preferably compliant, soft, comfortable, compressible and resilient to enhance body fit and comfort of the primary absorbent member. Preferably, the absorbent core 34 is compressible such that the primary absorbent member will deform under relatively small forces that are experienced during normal use. In addition to being compressible, the materials of the absorbent core 34 need to be flexible and conformable such that the primary absorbent member is able to provide improved fit into and around the labia and perineum by following the fluid bubble 31. The ability to follow the topography of the yielding fluid bubble 31 will provide intimate contact with the exposed genitalia of the female user. Intimate contact with the exposed female genitalia helps provide better fluid transfer from the user into the primary absorbent member without allowing fluid to bypass and/or run-off the primary absorbent member. While these characteristics of the absorbent core 34 allow for improved fit, they also improve the product to be both soft and comfortable for the wearer.

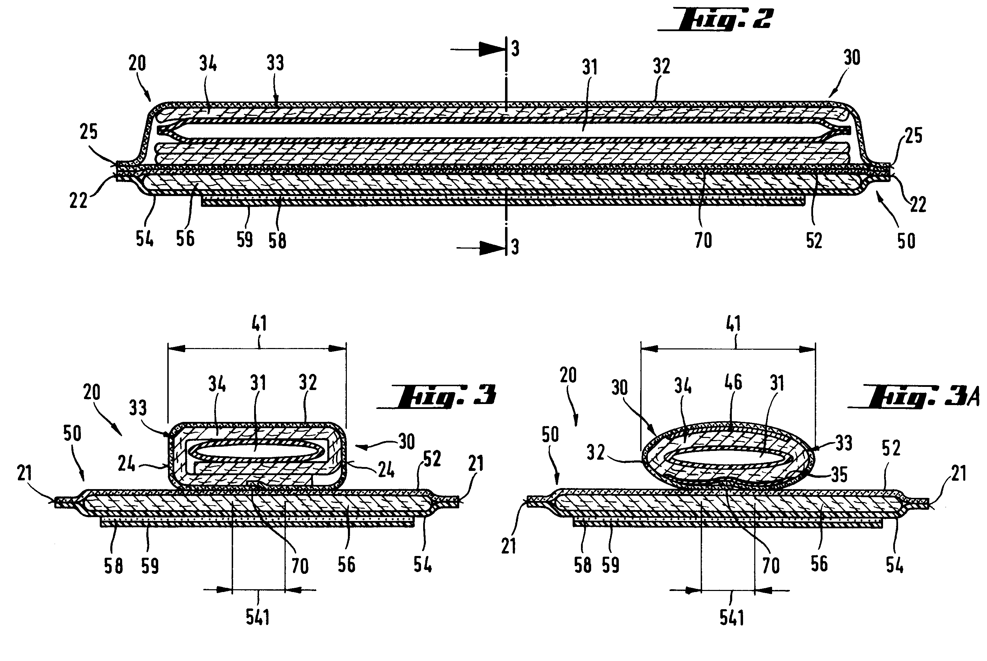

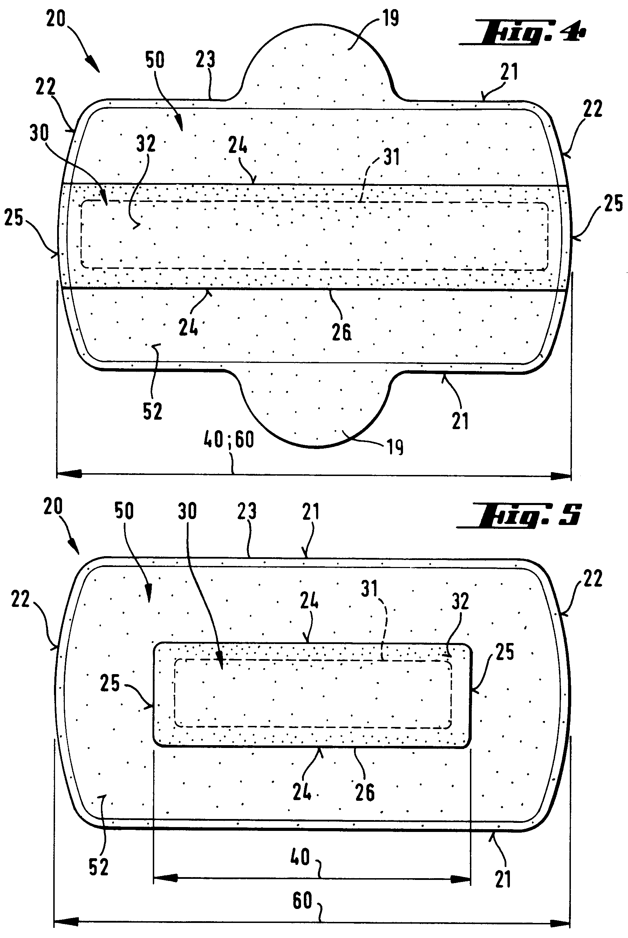

In the embodiment illustrated in FIGS. 2 and 3, the absorbent core 34 is comprised of an absorbent layer made of a thermally bonded airlay material longitudinally folded twice on itself in order to comprise the fluid bubble 31 internally.

The fluid bubble can comprise essentially any fluid, e.g. a gel, which is capable of providing the yielding effect; preferably the fluid is a gas and most preferably it is air. The skin of the bubble 31 can be made from any material which provides a sustainable internal amount of fluid over the expected product lifetime from manufacturing of the absorbent product to the disposal thereof. The material used for the skin of the bubble should be soft and compliant in order to allow the deformation or yielding of the bubble. Typical materials can be those also used for impervious backsheets of the secondary absorbent member 50 described hereinafter as well as polyethylene terephtalate film and aluminium film or mixtures and laminates of these materials.

The fluid bubble is typically longer along the longitudinal axis than wide along the lateral axis. Furthermore the bubble may have a variety of shapes which are typically symmetrical to the longitudinal axis but asymmetrical to the lateral axis. The function of the bubble may also be provided by a plurality of smaller individual bubbles which in combination provide the desired shape. In particular 2 or 3 bubbles may be used instead of a single bubble in order to raise the body facing surface of the compound sanitary napkin and to provide folding lines between the bubbles.

In one preferred embodiment shown in FIGS. 1 to 3 the fluid bubble 31 is comprised entirely within the primary absorbent member 30, but in any case it is preferred that the fluid bubble or the plurality of smaller bubbles does not extend beyond the periphery 26 of the primary absorbent member 30. As illustrated in FIGS. 1 and 2 the fluid bubble 31 is about the same length as the primary absorbent member 30; however, it is quite possible for the fluid bubble 31 to be shorter than the primary absorbent member 30 and still work effectively. It is preferred that the fluid bubble 31 is positioned so that, in use, it corresponds to the discharge area of the user; a fluid bubble that is shorter than the primary absorbent member might extend, for example, substantially along the central region 28 of the sanitary napkin.

The fluid bubble 31 is typically filled such that the filling volume is substantially below its maximum filling volume in order to provide a wobbly, flexible and easily deformable outer surface. This ability to deform, also referred to as yielding, provides the desired exceptional ability to adapt the surface topography of the bubble and the overlying absorbent means 33 and topsheet 32 to the topography of the individual wearer of a sanitary napkin according to the present invention. By filling the bubble no more than 90%, preferably no more than 80% of the maximum volume of the bubble it is possible to ensure that even under the temperature changes common during the wearing of such a product the fluid bubble will continue to be yielding such that it adapts the product to the wearer's topography.

As an alternative the material forming the skin of the fluid bubble at least on its body facing side may be elastic in order to provide the fluid bubble with the desired yielding characteristic. The filling of the bubble in this case can exceed 100 % or even 150 % of the unstretched bubble volume.

Referring now to FIG. 3A, there is shown a cross-sectional view taken along the transverse axis of another embodiment of a compound sanitary napkin of the present invention. In this embodiment, the absorbent core 34 is preferably comprised of airfelt. The airfelt absorbent core is manufactured in a generically oval shape to provide the primary absorbent member 30 with a generally oval shape; the fluid bubble 31 is comprised within the airfelt absorbent core 34. While the core 34 shown in FIGS. 3 and 3A has a generally rectangular or oval cross-section, the absorbent core may be manufactured in a wide variety of shapes such as circular, triangular, square, pentagonal, U-shaped, Z-folded, etc.

Optionally, the primary absorbent member 30 can comprise a fluid barrier 35. The fluid barrier 35 tends to contain absorbed fluids within the absorbent core 34 and can be constructed from materials having the same properties as the liquid impervious backsheet on the secondary absorbent member 50 described hereinafter.

Optionally, the primary absorbent member 30 may comprise an acquisition layer 46 positioned between the topsheet 32 and the absorbent core 34. The acquisition layer 46 may serve several functions including improving wicking of exudates over and into the absorbent core 34. By improving the wicking of exudates, the acquisition layer provides a more even distribution of the exudates throughout the absorbent core 34. The acquisition layer 46 may be comprised of several different materials including nonwoven or woven webs of synthetic fibers including polyester, polypropylene, or polyethylene; natural fibers including cotton or cellulose; blends of such fibers; or any equivalent materials or combinations of materials. Examples of sanitary napkins having an acquisition layer and a topsheet are more fully described in U.S. Pat. No. 4,950,264 issued to Osborn and U.S. Pat. Application Serial No. 07/810,774, "Absorbent Article Having Fused Layers", filed December 17, 1991 in the names of Cree, et al. In a preferred embodiment, the acquisition layer 46 may be joined with the topsheet by any of the conventional means for joining webs together, most preferably by fusion bonds as is more fully described in the above-referenced Cree application.

While the primary absorbent member can be generally of any cross-sectional shape in its unstressed condition it is preferably rectangular or oval in cross-section. The length 40 and the width 41 of the primary absorbent member 30 can be of any convenient dimension. The primary absorbent member 30 is preferably from about 2 to 35 cm long, more preferably from about 10 to 35 cm long, and most preferably from about 20 to 35 cm long. A particularly preferred primary absorbent member 30 has a length of about 24 cm.

One major function of the primary absorbent member 30 is to absorb and contain bodily fluids. It works in combination with the fluid bubble 31 that raises the body facing surface of the primary absorbent member 30 towards the discharge area of the user and, at the same time, due to its yielding capability, is able to deform and to adapt the surface topography of the primary absorbent member 30 to the topography of the wearer. In addition, the primary absorbent member 30 is preferably sized and shaped such that it will fit within the labia. Accordingly, the width and/or diameter of the primary absorbent member should be sized such that it will reside at least partially within the labia. That is, a portion of the primary absorbent member will preferably fit within the labia during use. Since the exposed female genitalia, including the labia, are generally referred to as soft body tissue, it is important that the materials comprising and the primary absorbent member be comfortable and relative soft such that they are non-irritating and/or uncomfortable for the user.

The primary absorbent member 30 is preferably from about 0.5 to 5 cm wide, more preferably from about 0.5 to about 4 cm wide, and most preferably from about 0.5 to about 3 cm wide. It has been found that a primary absorbent member having a width between 1.5 and 2.5 cm, preferably about 1.5 cm, is able to comfortably fit within at least a portion of the labial groove of most women.

Referring to FIGS. 1-3A, the second necessary constituent of the compound sanitary napkin of the present invention is the secondary absorbent member 50. The secondary absorbent member 50 preferably comprises a liquid permeable topsheet 52, a liquid impervious backsheet 54 joined with the topsheet 52, and an absorbent element 56 positioned between the topsheet 52 and the backsheet 54.

The topsheet 52 can be any fluid pervious material commonly used in sanitary napkins, disposable diapers, and the like. It can be any of the materials described above as being useful in the topsheet 32 of the primary absorbent member 30. The absorbent element 56 can be any absorbent material commonly used in sanitary napkins, disposable diapers, and the like. It can be any of the materials described above as being useful in the absorbent core 34 of the primary absorbent member 30.

As a practical matter, most of the bodily fluids are absorbed by and are contained within the absorbent core 34 of the primary absorbent member. One major function of the secondary absorbent member 50 is to protect the user's garments from soiling by absorbed fluids which may be expelled from the primary absorbent member or which may inadvertently bypass the primary absorbent member. Because the absorbent element 56 of the secondary absorbent member 50 performs a different function from that of the absorbent core 34, the absorbent element 56 can be, and most preferably is, thinner and less bulky than the absorbent core 34.

Optionally, the secondary absorbent member may be manufactured without an absorbent element. Since most if not all of the bodily fluids are preferably absorbed by and are contained within the absorbent core of the primary absorbent member, the secondary absorbent member 50 need only to protect the user's garments from soiling by relatively small amounts of fluids. Accordingly, an absorbent element may not be necessary to contain the fluids within the secondary absorbent member in order to prevent them from soiling the user's garments.

The backsheet 54 is impervious to liquids (e.g., menses and/or urine) and is preferably manufactured from a thin plastic film, although other flexible liquid impervious materials may also be used. In use, the backsheet 54 is interposed between the absorbent element 56 and the user's undergarments. The function of the backsheet 54 is to prevent exudates which may be expelled from or which inadvertently bypass the primary absorbent element and exudates absorbed and contained in the absorbent element 56 from contacting and soiling the user's undergarments. The backsheet 54 may thus comprise a woven or nonwoven material, polymeric films such as thermoplastic films of polyethylene or polypropylene, semi-permeable films which provide breathability but prevent liquid transport, or composite materials such as a film-coated nonwoven material. Preferably, the backsheet is a polyethylene film having a thickness of from about 0.012 mm (0.5 mil) to about 0.015 mm (2.0 mil). The backsheet is preferably embossed and/or matte finished to provide a more clothlike appearance. Further, the backsheet may permit vapors to escape from the absorbent element 56 (i.e., breathable) while still preventing exudates from passing through the backsheet.

Preferably, the secondary absorbent member 50 is provided with a support means or attachment means, such as adhesive attachment means 58. The adhesive attachment means 58 provides a means for securing the compound sanitary napkin 20 in the crotch portion of the user's undergarment or panty. The adhesive is typically covered with a removable release liner 59 in order to keep the adhesive from drying out or adhering to a surface other than the crotch portion of the panty prior to use. Any commercially available release liners commonly used for such purposes can be utilized herein. The compound sanitary napkin 20 of the present invention is used by removing the release liner 50 and thereafter placing the sanitary napkin in a panty so that the adhesive 58 contacts the panty. The adhesive 58 maintains the sanitary napkin in its position within the panty during use.

As shown in FIGS. 1-3, the secondary absorbent member can be of generally rectangular shape. Other suitable shapes include but are not limited to oval, hourglass, dog-bone, asymmetric, etc.

Referring to FIG. 1, the secondary absorbent member 50 preferably has a length 60 and a width 61. The secondary absorbent member is preferably from about 20 to 40 cm long, more preferably from about 25 to 35 cm long, and most preferably is about 30 cm long.

While it can be of generally any cross-section in its unstressed condition, the secondary absorbent member is preferably rectangular in cross-section. The secondary absorbent member is preferably from about 5 to 15 cm in width, more preferably from about 5 to 10 cm in width, and most preferably from about 5 to 8 cm in width. The thickness of the secondary absorbent member 50, as shown in cross-section in FIGS. 2 and 3, is generally somewhat less than its width.

Preferably, the secondary absorbent member will be thin and have a caliper of less than about 3.0 millimeters, more preferably less than about 2.6 millimeters, more preferably less than about 2.2 millimeters, and most preferably less than about 2.0 millimeters.

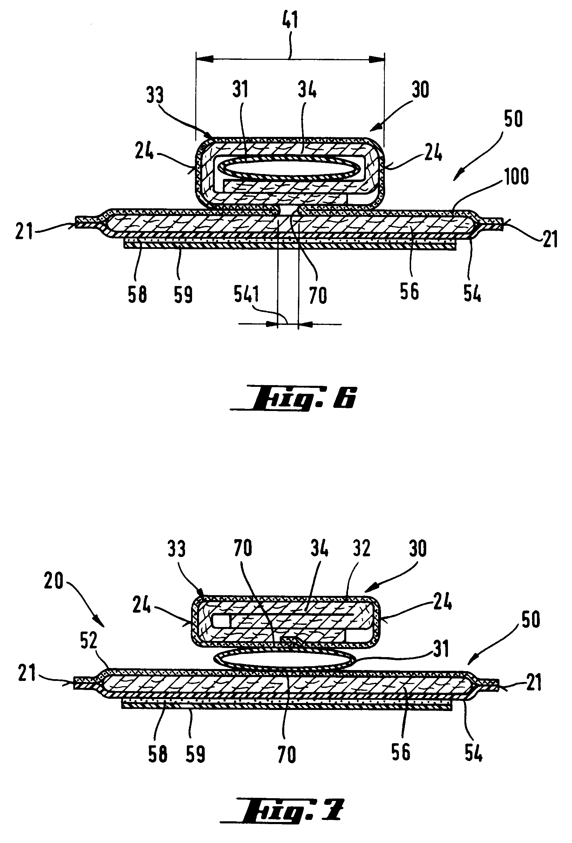

Optionally, the secondary absorbent member 50 may have two flaps 19 each of which are adjacent to and extend laterally from the side edge of the absorbent core, as shown in FIG. 4. The flaps 19 are configured to drape over the edges of the wearer's panties in the crotch region so that the flaps are disposed between the edges of the wearer's panties and the wearer's thighs. The flaps serve at least two purposes. First, the flaps help serve to prevent soiling of the wearer's body and panties by menstrual fluid, preferably by forming a double wall barrier along the edges of the panty. Second, the flaps are preferably provided with attachment means on their garment facing surface so that the flaps can be folded back under the panty and attached to the garment facing side of the panty. In this way, the flaps serve to keep the sanitary napkin properly positioned in the panty.

In a preferred embodiment, the flaps are comprised of the topsheet, absorbent element, and backsheet. Further, the flaps are preferably unitary to the laminae of the secondary absorbent element. In other words, the topsheet, absorbent element, and backsheet simply extend laterally outward to form the flaps. However, the flaps need not be unitary with the secondary absorbent member, but can be separate elements which are affixed to the secondary absorbent member. Further, the flaps can be comprised of a single substrate or other laminae configurations. It is recommended, however, that the flaps have a liquid impervious backsheet to prevent exudates which reach the flaps from soiling the edges of the wearer's panties.

A number of sanitary napkins having flaps suitable or adaptable for use with the secondary absorbent member 50 of the compound sanitary napkin 20 of the present invention are disclosed in U.S. Pat. No. 4,687,478 issued to Van Tilburg on Aug. 18, 1987; U.S. Pat. No. 4,589,876 issued to Van Tilburg on May 20, 1986; and U.S. Pat No. 4,608,047 issued to Mattingly on Aug. 26, 1986.

Optionally, the secondary absorbent member may comprise components that naturally wrap the sides of a wearer's panties. A sanitary napkin having components that naturally wrap the sides of a wearer's panties suitable for use with the secondary absorbent member of the compound sanitary napkin 20 of the present invention are disclosed in U.S. Patent Application Serial No. 08/096,121, (P&G Case 4961) entitled "Absorbent Article having Panty Covering Components that Naturally Wrap the Sides of Panties", filed July 22, 1993, in the names of Lavash, et al and U.S. Patent Application Serial No. 08/277733 (P&G Case 5354) entitled "Absorbent Articles Having Undergarment Covering Components with Zones of Extensibility", filed July 20, 1994, in the names of Weinberger, et al.

Preferably, the minimum width of the secondary absorbent member is at least 1.5 times the maximum width of the primary absorbent member. More preferably, the minimum width of the secondary absorbent member is at least 2 times the maximum width of said primary absorbent member. Most preferably, the minimum width of the secondary absorbent member is in the range from about 3 to about 8 times the maximum width of the primary absorbent member.

Preferably, the secondary absorbent member is about the same length as the primary absorbent member while the compound sanitary napkin is in an unstressed condition. However, it is quite possible for the secondary absorbent member to be longer than the primary absorbent member and still function effectively.

The individual components of the primary absorbent member 30 and the secondary absorbent member 50 may be comprised of components that are extensible or elastically stretchable in the longitudinal and/or lateral direction when the compound sanitary napkin is worn.

Referring now to FIG. 1, it can be seen that the primary absorbent member 30 and the secondary absorbent member 50 have their length each parallel, and in fact corresponding, to the longitudinal axis of the sanitary napkin 20.

To form the compound sanitary napkin of the present invention, the primary absorbent member and the secondary absorbent member are affixed by joining means generally indicated as 70 in FIGS. 2, 3 and 3A. The precise nature of the joining means is immaterial so long as the joining means selected serves to join the primary absorbent member and the secondary absorbent member into the compound sanitary napkin of the present invention with sufficient tenacity that the primary absorbent member and the secondary absorbent member are not disconnected during use. Joining means such as adhesive attachment with well known hot melt and pressure sensitive adhesives are quite satisfactory. If the nature of the components selected to construct the constituents of the compound sanitary napkin so permit, heat welding, ultrasonic welding, or a combination of both heat and ultrasonic welding can be used.

The primary absorbent member 30 may be affixed to the secondary absorbent member by joining means in such a manner that the affixed length is at least 25% of the length of the primary absorbent member 30, preferably the affixed length is at least 50% of the length of the primary absorbent member 30, more preferably 75%; even more preferably, the primary absorbent member 30 is affixed to the secondary absorbent member 50 by joining means 70 extending along substantially the entire length of the primary absorbent member 30.

The primary absorbent member 30 has a width 41. The compound sanitary napkin has a joining means width 541. The joining means width 541 is less than the width 41 of the primary absorbent member 30. Preferably, the joining means width 541 is less than 75% of the width of the primary absorbent member 30. More preferably, the joining means width 541 is less than 50% of the width of the primary absorbent member 30. Most preferably, the joining means width 541 is less than 25% of the width of the primary absorbent member 30.

Preferably, the primary absorbent member exhibits a "stationary resistance" sufficient enough to provide enhanced performance. As used herein, the term "stationary resistance" refers to the resistance exhibited by the primary absorbent member to forces applied to the primary absorbent member within the central region such that the side edges of the primary absorbent member do not extend beyond the side edges of the secondary absorbent member. In other words, the stationary resistance describes the relative movement of the primary absorbent member compared to the secondary absorbent member. It is preferred that the side edges of the primary absorbent member do not extend beyond the side edges of the secondary absorbent member even under typically in use forces. By keeping the side edges of the primary absorbent member within the side edges of the secondary absorbent member, under typically in use forces, the opportunity for fluid to bypass or be expelled from the primary absorbent member and onto a surface other than the secondary absorbent member, for example, the user's skin or undergarments, is substantially reduced.

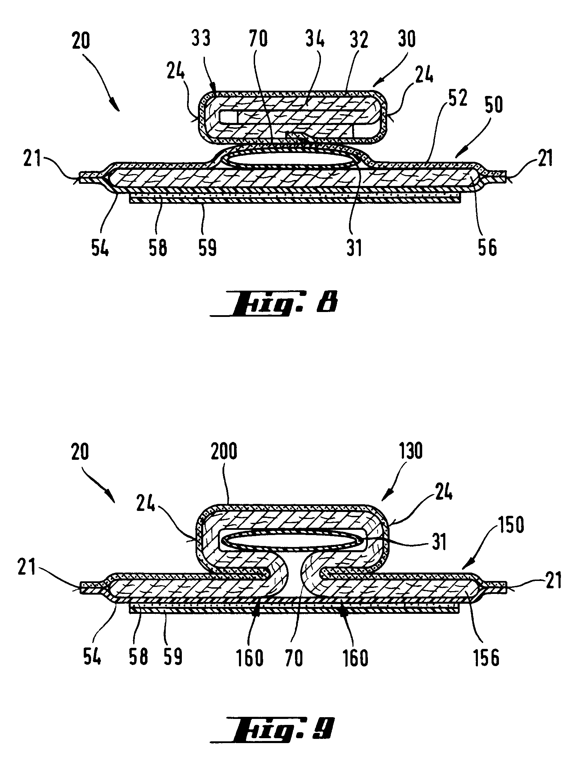

Referring now to FIG. 5, there is shown another preferred embodiment of a compound sanitary napkin 20 of the present invention. The primary absorbent member 30 has a length 40 that is less than the length 60 of the secondary absorbent member 50; the fluid bubble 31 will have accordingly a length not greater than the length of the primary absorbent member 30. Any other element of this embodiment is similar to what has been already described referring to FIGS. 1 to 3.

In FIG. 3 it can be seen that topsheet 32 completely encases the absorbent core 34 of the primary absorbent member 30. In this embodiment, the topsheet 32 for the primary absorbent member 30 is separate and distinct from the topsheet 52 for the secondary absorbent member 50.

Optionally, the topsheet for the primary absorbent member 30 and the secondary absorbent member 50 may be made of a single web of material, such as topsheet 100 as seen in FIG. 6. In this embodiment topsheet 100 is used for the topsheet on both the primary absorbent member 30 and the secondary absorbent member 50. In the embodiment of FIG. 6 the topsheet 100 can serve as a joining means 70 connecting the primary absorbent member and the secondary absorbent member together. The compound sanitary napkin may also include additional joining means to connect the primary absorbent member to the secondary absorbent member. The joining means width 541 is less than the width 41 of the primary absorbent member 30.

In FIG. 7 a further embodiment of the present invention is shown, in which the fluid bubble 31 is comprised between the primary absorbent member 30 and the secondary absorbent member 50 of the sanitary napkin 20; in this case the joining means 70 that affix the primary absorbent member 30 to the secondary absorbent member 50 may also comprise the fluid bubble 31, which is preferably affixed to the secondary absorbent member 30 along its entire length, if the fluid bubble 31 has the same length of the primary absorbent member 30. Otherwise, if the fluid bubble 30 is shorter than the primary absorbent member 31 and extends for example substantially along the central region of the sanitary napkin, the primary absorbent member 31 may be joined at its ends directly to the secondary absorbent member 50, while being fully, partially or not at all affixed to the fluid bubble 31.

FIG. 8 shows another preferred embodiment of the present invention in which the fluid bubble 31 is comprised within the secondary absorbent member 50, more precisely between the topsheet 52 and the absorbent element 56 of the secondary absorbent member 50. Alternatively, the fluid bubble 31 can also be provided within the absorbent core 56 of the secondary absorbent member 50 or between backsheet 54 and absorbent core 56 of the secondary absorbent member 50.

Referring now to FIG. 9, there is shown another preferred embodiment of the present invention. The primary absorbent member 131 and the secondary absorbent member 150 are integrally made from a single absorbent element 156 by means of two Z-shaped folds 160 parallel to the longitudinal axis of the sanitary napkin 20; topsheet 200 is used for the topsheet on both the primary absorbent member 130 and the secondary absorbent member 150. In the embodiment of FIG. 9 the absorbent element 156 and the topsheet 200 can serve as joining means 70 connecting the primary absorbent member 130 and the secondary absorbent member 150 together. As shown in FIG. 9 the fluid bubble 31 can be encased by the upper part of the 2-fold of the absorbent element 156.

## Claims

1. A compound disposable absorbent article for wearing adjacent a body discharge area, said article having a body facing surface, a garment facing surface, a longitudinal axis and a lateral axis, a primary absorbent member having a length and a width and a secondary absorbent member having a length and a width, said primary absorbent member and said secondary absorbent member having their length each parallel to said longitudinal axis, said secondary absorbent member being closer to said garment facing surface than said primary absorbent member, said primary absorbent member being affixed to said secondary absorbent member by joining means, characterised in that said article further comprises a yielding fluid bubble to raise said body facing surface towards said discharge area.

2. A compound disposable absorbent article according to claim 1, characterized in that said fluid bubble is a gas bubble, and preferably an air bubble.

3. A compound disposable absorbent article according to any preceding claim, characterized in that said fluid bubble is longer along said longitudinal axis than along said lateral axis and does not extend beyond the periphery of said primary absorbent member.

4. A compound disposable absorbent article according to any preceding claim, characterized in that said fluid bubble is positioned within said secondary absorbent member.

5. A compound disposable absorbent article according to any preceding claim, characterized in that said fluid bubble is positioned within said primary absorbent member.

6. A compound disposable absorbent article according to any preceding claim, characterized in that said width of said secondary absorbent member is equal or greater than said width of said primary absorbent member.

7. A compound disposable absorbent article according to any preceding claim, characterized in that the minimum width of said secondary absorbent member is at least 1.5 times, and preferably from 3 to 8 times, the maximum width of said primary absorbent member.

8. A compound disposable absorbent article according to any preceding claim, characterized in that the maximum width of said primary absorbent member is less than 3 centimetres.

9. A compound disposable absorbent article according to any preceding claim, characterized in that said primary absorbent member is affixed to said secondary absorbent member by joining means and the affixed length is at least 25% of said length of said primary absorbent member along said longitudinal axis, preferably is at least 50% and most preferably said joining means extend substantially continuously along said length of said primary absorbent member.

10. A compound disposable absorbent article according to any preceding claim, characterized in that said primary absorbent member and said secondary absorbent member are integrally made from a single absorbent element, preferably by means of two Z-shaped folds parallel to said longitudinal axis of said article.
